# EUROPÄISCHE PATENTSCHRIFT

(11) **EP 1 027 928 B1**
(45) Veröffentlichungstag und Bekanntmachung des Hinweises auf die Patenterteilung: **01.03.2006**
(21) Anmeldenummer: 00102083.3
(22) Anmeldetag: 03.02.2000
(51) Int. Cl.: B01J 23/745, B01J 23/76, B01J 37/34, C07C 5/333, C07C 15/067

(54) **Katalysator zur Dehydrierung von Ethylbenzol zu Styrol**
Catalyst for dehydrogenation of ethylbenzene to styrene
Catalyseur pour la déshydrogénation d'éthylbenzène en styrène

(30) Priorität: 10.02.1999 DE 19905392; 10.07.1999 DE 19932362
(43) Veröffentlichungstag der Anmeldung: 16.08.2000
(73) Patentinhaber: BASF Aktiengesellschaft, 67056 Ludwigshafen (DE)
(72) Erfinder: Baier, Michael Dr., 68161 Mannheim (DE); Petersen, Hermann Dr., 67269 Grünstadt (DE); Wanjek, Herbert Dr., 67133 Maxdorf (DE); Walsdorff, Christian Dr., 67061 Ludwigshafen (DE)

(56) Entgegenhaltungen:
- EP-A- 0 850 881
- EP-A- 0 894 528
- PATENT ABSTRACTS OF JAPAN vol. 010, no. 239 (C-367), 19. August 1986 (1986-08-19) & JP 61 072601 A (NIPPON MINING CO LTD), 14. April 1986 (1986-04-14)

## Beschreibung

Die Erfindung betrifft einen Katalysator, enthaltend Eisenoxid, die einen Restgehalt an Chlor von 800 bis 1500 ppm aufweisen, ein Verfahren zu dessen Herstellung sowie ein Verfahren zur Dehydrierung von Ethylbenzol zu Styrol in Gegenwart des Katalysators.

Zur Herstellung von Styrolkatalysatoren auf Basis Fe₂O₃ und K₂O werden in der Regel natürliche und synthetische Eisenoxide, wie α-FeOOH, α-Fe₂O₃, γ-Fe₂O₃ und Fe₃O₄ verwendet. Die synthetischen Eisenoxide werden in der Regel durch Fällen von Eisensalzlösungen und thermische Zersetzung hergestellt.

Neben den üblichen Eisenoxiden werden auch spezielle Eisenoxide oder modifizierte Eisenoxide als Eisenkomponente zur Herstellung von Dehydrierkatalysatoren beschrieben.

Die zur Herstellung der Dehydrierkatalysatoren nach EP-A 0 532 078 eingesetzte eisenhaltige Verbindung enthält 10 bis 100 Gewichtsprozent eines glimmerartigen Eisenoxids mit einer bevorzugten maximalen Plättchengröße von weniger als 100 µm.

Die US 5,023,225 beschreibt die Verwendung von Chrom-modifiziertem Eisenoxid zur Herstellung von Dehydrierkatalysatoren. Das rote Eisenoxid wird durch Mischen von gelbem Eisenhydrat mit Chromoxid oder einem Chromsalz und Erhitzen der Mischung hergestellt.

Die WO 96/18593 beschreibt einen eisenhaltigen Dehydrierkatalysator, dessen Eisenkomponente im Katalysator eine bimodale Porengrößenverteilung aufweist. Bevorzugt werden magnetische Eisenoxidverbindungen, wie Magnetit, eingesetzt.

Ein vorbehandeltes ("predoped") Eisenoxid wird nach WO 96/18458 verwendet. Die Vorbehandlung erfolgt mit einer Vorbehandlungssubstanz, die ein Element ausgewählt aus der Gruppe Be, Mg, Ca, Sr, Ba, Sc, Ti, Zr, Hf, V, Ta, Mo, W, Mn, Tc, Re, Ru, Os, Co, Rh, Ir, Ni, Pd, Pt, Cu, Ag, Au, Zn, Cd, Hg, Al, Ga, In, Tl, Ge, Sn, Pb, Bi, Ce, Pr, Nd, Pm, Sm, Eu, Gd, Tb, Dy, Ho, Er, Tm, Yb und Lu, enthält und Erhitzen der Eisenoxidmischung auf mindestens 600°C. Anschließend erfolgt die Katalysatorformung.

Die WO 96/18594 beschreibt einen Eisenoxidkatalysator mit Eisenoxidteilchen, die eine mittlere längste Abmessung im Bereich von 2 bis 10 µm besitzen. Die mittleren Porendurchmesser liegen zwischen 0,22 und 0,30 µm, die Porenvolumen zwischen 0,16 und 0,22 cm³/g. Zur Herstellung wurde ein durch Dehydratisierung eines gelben α-Fe(OOH)-Zwischenproduktes aus Eisenspänen hergestelltes Eisenoxid α-Fe(OOH) verwendet (Penniman Eisenoxid).

Die WO 96/18457 beschreibt ein Verfahren zur Herstellung von Eisenoxidkatalysatoren, bei denen das Eisenoxid vor dem Mischen mit ein oder mehreren Promotoren zu Eisenoxidteilchen mit einer BET-Oberfläche von weniger als 1,9 m²/g, einer Partikellänge von 0,3 - 3 µm und einer Partikelbreite von 0,2 - 2 µm umstrukturiert wird. Als Umstrukturierungsagens werden Verbindungen der Elemente Mo, Cu, Ca, Zn, Mn, Sn, Ti, Bi, Co, Ce, W, Cr, Mg, und V verwendet.

Die genannten Katalysatoren benötigen als Rohstoffe Eisenoxide, die eine aufwendige Herstellung oder Modifizierung verlangen.

Aufgabe der vorliegenden Erfindung war es daher, den genannten Nachteilen abzuhelfen und einen preisgünstigen und einfach herzustellenden Katalysator zur Dehydrierung von Ethylbenzol zu Styrol zu finden. Der Katalysator sollte insbesondere ein hohes Porenvolumen bei gleichzeitig hoher Aktivität, Selektivität und hoher mechanischer Stabilität aufweisen.

Demgemäß wurde ein Katalysator, enthaltend Eisenoxid und 5 bis 40 Gew.-% Kalium, berechnet als K₂O, gefunden, wobei zur Herstellung des Katalysators ein Eisenoxid, das durch Sprühröstung einer Eisensalzlösung erhalten wurde, eingesetzt wird, wobei das Eisenoxid einen Restgehalt an Chlor von 800 bis 1500 ppm aufweist.

Bevorzugt wird das eingesetzte Eisenoxid durch Sprühröstung einer salzsauren, eisenchloridhaltigen Lösung gewonnen.

Für die Herstellung der erfindungsgemäßen Katalysatoren eignet sich ein durch Sprühröstung hergestelltes Eisenoxid mit einem Stampfgewicht im Bereich von 0,6 bis 1, bevorzugt 0,6 bis 0,8 g/cm³ und einer spezifischen Oberfläche im Bereich von 1 bis 10, bevorzugt 2 bis 7, besonders bevorzugt 3 bis 5 m²/g.

Das Porenvolumen der erfindungsgemäßen Katalysatoren beträgt mindestens 0,2 cm³/g und liegt bevorzugt im Bereich von 0,25 bis 0,5 cm³/g. Der mittlere Porendurchmesser (Medianwert) beträgt mindestens 0,3 µm und liegt bevorzugt im Bereich von 0,4 bis 0,8 µm.

Die erfindungsgemäßen Katalysatoren enthalten zusätzlich zum Eisenoxid mindestens eine Kaliumverbindung. Als Kaliumverbindung werden bevorzugt Kaliumcarbonat, Kaliumhydroxid oder Kaliumoxalat eingesetzt. Der Katalysator enthält 5 bis 40 Gew.-% Kalium, berechnet als K₂O.

Weiterhin kann der Katalysator einen oder mehrere der üblichen Promotoren zur Steigerung von Selektivität, Aktivität oder Stabilität in üblichen Konzentrationen enthalten. Als Promotoren eignen sich Verbindungen von Elementen ausgewählt aus der Gruppe Be, Mg, Ca, Sr, Ba, Sc, Ti, Zr, Hf, V, Ta, Mo, W, Mn, Tc, Re, Ru, Os, Co, Rh, Ir, Ni, Pd, Pt, Cu, Ag, Au, Zn, Cd, Hg, Al, Ga, In, Tl, Na, Cs, La, Li, Ge, Sn, Pb, Bi, Ce, Pr, Nd, Pm, Sm, Eu, Gd, Tb, Dy, Ho, Er, Tm, Yb und Lu, die einzeln oder in Mischungen verwendet werden können. Bevorzugte Promotoren sind Verbindungen ausgewählt aus der Gruppe Mg, Ca, Ce, V, Cr, Mo, W, Ti, Mn, Co und A1. Besonders bevorzugte Promotoren sind Mg, Ca, Ce, V, Cr, Mo und W. Die Katalysatoren können einen, bevorzugt zwei, insbesondere drei oder mehr Promotoren aus der Gruppe Mg, Ca, Ce, V, Cr, Mo, W enthalten. Die Promotoren werden bevorzugt in Mengen von jeweils 0 bis 15, insbesondere 1 bis 10 Gew.-%, berechnet als stabilste Oxide, zugesetzt.

Die erfindungsgemäß eingesetzten Eisenoxide können auch ganz oder teilweise die Kaliumverbindung oder die genannten Promotoren enthalten. Hierzu kann beispielsweise ein Teil der Kaliumverbindung und/oder ein Teil der Promotoren der zur Sprühröstung verwendeten Eisensalzlösung zugesetzt werden.

Die erfindungsgemäßen Katalysatoren können nach den bekannten ein- oder mehrstufigen Herstellungsverfahren erhalten werden, beispielsweise wie in EP-A 0 195 252 oder EP-A 0 866 731 beschrieben. Hierzu kann das durch Sprühröstung einer Eisensalzlösung erhaltene Eisenoxid alleine oder zusätzlich zu den üblichen natürlichen oder synthetischen Eisenoxiden, wie α-FeOOH, γ-FeOOH, α-Fe₂O₃, γ-Fe₂O₃ oder Fe₃O₄, eingesetzt werden. Der Anteil des durch Sprühröstung einer Eisensalzlösung erhaltenen Eisenoxides beträgt 10 bis 100 Mol.-%, bevorzugt 50 bis 100 Mol.-%, bezogen auf alle eingesetzten Eisenverbindungen.

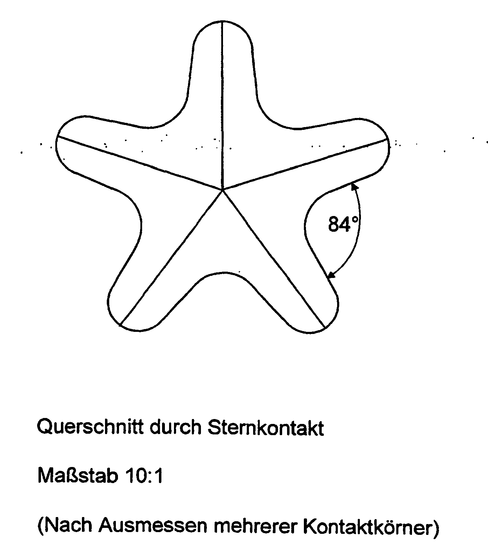

Die feinpulvrigen Katalysatorbestandteile können trocken gemischt oder in Wasser suspendiert und sprühgetrocknet werden. Das trokkene Pulver wird anschließend zu mechanisch stabilen Formkörpern tablettiert oder unter Zugabe von Wasser zu einer pastösen Masse angeteigt und zu Strängen extrudiert und abgelängt. Hierbei können Verformungshilfsmittel wie Stearate, Walocel, Graphit oder Stärke eingesetzt werden. Bevorzugt wird die Katalysatormasse zu Voll- oder Hohlsträngen mit einem Durchmesser von 2,5 bis 6 mm und einer Länge von 5 bis 50 mm verformt. Besonders bevorzugt werden Stränge mit sternförmigem Querschnitt, wie in Figur 1 abgebildet, und 3-mm-Vollstränge hergestellt.

Die Extrudate werden anschließend kontinuierlich oder diskontinuierlich bei Temperaturen in der Regel im Bereich von 80 bis 140°C getrocknet. Die getrockneten Formkörper können anschließend einstufig oder mehrstufig bei Temperaturen im Bereich von 200 bis 1000°C getempert und/oder calciniert werden.

Die erfindungsgemäßen Katalysatoren eignen sich zur nicht-oxidativen Dehydrierung von Kohlenwasserstoffen, insbesondere zur Dehydrierung von Ethylbenzol zu Styrol. Hierbei wird im allgemeinen ein Gemisch aus Wasserdampf und Ethylbenzol im molaren Verhältnis im Bereich von 2 bis 20, bevorzugt im Bereich von 5 bis 15, und bei Temperaturen im Bereich von 500 bis 700°C über den Katalysator geleitet. Beim adiabaten Verfahren beträgt die Temperatur am Reaktoreingang in der Regel 600 bis 650°C und sinkt infolge der Endothermie der Reaktion auf 530 bis 570°C ab. Bevorzugt wird die Reaktion bei Atmosphärendruck oder darunter durchgeführt.

Die erfindungsgemäßen Katalysatoren weisen ein großes Porenvolumen auf. Dadurch wird das Litergewicht des Katalysator auf unter 1,3 kg/l, gemessen an 3 mm-Vollstrangkatalysatoren verringert, ohne die Aktivität und Selektivität zu erniedrigen. Gleichzeitig ist die Schnitthärte mit über 20 N für den technischen Einsatz ausreichend.

### Beispiele

### Beispiel 1

900 g Eisenoxid Hoogovens RIO-200 (Fa. Hoogovens Staal BV) wurden zu einer Suspension aus 168 g K₂CO₃, 200 g Ce₂(CO₃)₃, 46 g CaCO₃, 29 g MoO₃ und 61 g basischem Magnesiumcarbonat (4MgCO₃xMg(OH)₃*4H₂O) in 4,3 l Wasser unter Rühren zugegeben. Die Sprühmaische wurde anschließend bei einem Feststoffgehalt von 25 Gew.-% sprühgetrocknet. Das erhaltenen Sprühpulver mit ca. 340 ml Wasser innerhalb 30 Minuten zu einer pastösen Masse verarbeitet und in einer Strangpresse zu zylindrischen Vollsträngen von 3 mm Durchmesser geformt und in ca. 10 mm lange Stücke geschnitten. Die Katalysatorstränge wurden anschließend in einem Umluftofen 1 Stunde bei 120°C getrocknet und in einem Calcinierofen zunächst 2 Stunden bei 300°C und dann 1 Stunde bei 875°C calciniert.

### Vergleichsversuche V1 bis V3

Beispiel 1 wurde wiederholt mit dem Unterschied, daß als Eisenoxid folgende synthetische Eisenoxide in gleichen Mengen eingesetzt wurden.

| | | | |
|---|---|---|---|
| V1: | gefälltes | α-Fe₂O₃: | Bayferrox 1360 |
| V2: | synthetisches | γ-Fe₂O₃: | Bayferrox E AB 21 bzw. |
| V3: | gefälltes | α-Fe₂O₃: | Bayferrox 720 N |

Die an den Katalysatorsträngen gemessenen Daten sind in Tabelle 1 zusammengestellt:

### Beispiel 2

900 g Eisenoxid Hoogovens Rio-250 (Fa. Hoogovens Staal BV) wurden unter Rühren zu einer Suspension aus 168 g Pottasche, 249 g Ce(CO₃)₃, 34 g Weißkalkhydrat, 35 g Ammoniumheptamolybdat und 26 g Magnesit in 4,3 1 Wasser zugegeben. Das erhaltene Sprühpulver wird mit ca. 500 ml Wasser innerhalb von etwa 30 min. zu einer pastösen Masse verarbeitet, zu zylindrischen Vollsträngen von 3 mm Durchmesser verformt und anschließend zu ca. 1 cm langen Stücken abgelängt. Die Katalysatorstränge wurden anschließend 1 h bei 120°C getrocknet und in einem Calcinierof en zunächst 2 Stunden bei 300°C und dann 1 Stunde bei 875°C calciniert.

### Vergleichsversuch V4

Beispiel 2 wurde wiederholt mit dem Unterschied, daß anstelle des Rio-250 Eisenoxids das Eisenoxid Bayferrox 1360 verwendet wurde.

Die Katalysatoren B1 und V1 bis V3 wurden am Katalysatorsplitt getestet. Dafür wurden Splitt-Fraktionen mit 0,5 bis 0,7 mm Teilchendurchmesser in Rohrreaktoren eingebaut, die durch Salzschmelzen isotherm beheizt wurden, und bei Normaldruck mit einer LHSV von 1,46/h und einem D/EB von 1,25 kg/kg getestet. Die zugehörigen Performance-Werte sind in Tabelle 2 wiedergegeben.

Die Katalysatoren B2 und V4 wurden außer am Splitt auch am Vollstrang getestet. In den Vollstrang-Tests wurden 250 ml des betreffenden Katalysators in einen isothermen Rohrreaktor mit 30 mm Innendurchmesser gefüllt. Die Katalysatoren wurden bei einem Druck von 0,4 bar mit einer LHSV von 0,45/h bei D/EB 1,25 kg/kg und D/EB 1,45 kg/kg getestet. Nach 10 Tagen hatten die Katalysatoren ein stabiles Umsatz- und Selektivitäts-Niveau erreicht und die Zusammensetzung des flüssigen und gasförmigen Austrags wurde bilanziert.

Das Stampfgewicht (Litergewicht) wurde gemessen mit einem Stampfvolumeter JEL der Fa. Engelsmann (Ludwigshafen). Vor der Messung der Stampfdichte wurden die Stränge 750 mal gestampft. Das Schüttgewicht ist das Gewicht vor dem Stampfen der Stränge. Die Porenvolumen wurden nach DIN-Norm 66133 bestimmt.

Der Kontaktwinkel des Quecksilbers bei der Bestimmung des mittleren Porendurchmessers betrug 140° (DIN. 66133).

Zur Bestimmung der Schnitthärte wurde mit einer Schneide von 0,3 mm eine zunehmende Belastung auf das Extrudat ausgeübt, bis dieses zerschnitten war (Gerät der Fa. Zwick (Ulm). Aus 25 Strängen wurde der Mittelwert gebildet.

**Tabelle 1: Eigenschaften der Katalysatoren**

| Katalysator | | B1 | V1 | V2 | V3 |
|---|---|---|---|---|---|
| Schüttgewicht | kg/l | 0,870 | 1,126 | 0,994 | 0,900 |
| Stampfgewicht | kg/l | 0,978 | 1,26 | 1,107 | 1,008 |
| BET-Oberfläche | m²/g | 3,4 | 2,6 | 6,8 | 6,9 |
| Porenvolumen | g/cm³ | 0,38 | 0,23 | 0,30 | 0,35 |
| Mittlere Porendurchmesser (Medianwert) | µm | 0, 65 | 0,38 | 0,20 | 0,30 |
| CeO₂-Kristallitgröße | nm | 24 | 24 | 23 | 25 |
| Schnitthärte | N | 27 | 75 | 30 | 34 |

**Tabelle 2:**

| Temp. | Katalysator | B1 | V1 | V2 | V3 |
|---|---|---|---|---|---|
| 560°C | Umsatz [%] | 27,9 | 29,1 | 25,3 | 24,7 |
| | Selektivität [%] | 97,7 | 97,7 | 97,4 | 97,7 |
| | Styrolanteil [Gew.-%] | 26,9 | 28,1 | 24,3 | 23,8 |
| 580°C | Umsatz [%] | 40,7 | 40,1 | 37,2 | 35,6 |
| | Selektivität [%] | 97,1 | 97,3 | 97,0 | 97,3 |
| | Styrolanteil [Mol.-%] | 39,1 | 38,7 | 35,7 | 34,3 |
| 600°C | Umsatz [%] | 54,5 | 50,8 | 48,1 | 50,3 |
| | Selektivität [%] | 96,2 | 96,4 | 96,5 | 96,4 |
| | Styrolanteil [Mol.-%] | 52,1 | 49 | 46,0 | 48,1 |
| 630°C | Umsatz [%] | 71,5 | 68,7 | 66,7 | 67,5 |
| | Selektivität [%] | 94,0 | 95,4 | 94,8 | 94,6 |
| | Styrolanteil [Mol.-%] | 67,4 | 65,6 | 63,3 | 63,8 |

Die Eigenschaften und Performance-Daten der Katalysatoren B1 und V2 sind in der Tabelle 3 zusammengefaßt.

**Tabelle 3: Eigenschaften und Performance der Katalysatoren B2 und V4**

| | | B2 | | V4 | |
|---|---|---|---|---|---|
| Schnitthärte | | 44N | | 57N | |
| Stampfgewicht | | 1,142 kg/l | | 1,304 kg/l | |
| BET-Oberfläche | | 2,9 m²/g | | 2,5 m²/g | |
| Porenvolumen | | 0,28 ml/g | | 0,22 ml/g | |
| Mittlerer Porendurchmesser | | 0,48 µm | | 0,40 µm | |
| Performance am Vollstrang | | | | | |
| H₂O (EB = 1,45 kg/kg; 0,4 bar: | | | | | |
| | U(600°C)/S (600°C) | 80,4 | %/94,8 % | 79,7 | %/94,7 % |
| U | (575°C)/S (575°C) | 69,8 | %/96,4 % | 65,8 | %/96,8 % |
| U | (550°C)/S (550°C) | 50,8 | %/97,7 % | 44,5 | %/97,9 % |
| H₂O/EB = 1,25 kg/kg; 0,4 bar: | | | | | |
| U | (575°C)/S (575°C) | 68,8 | %/96,1 % | 65,1 | %/96,6 % |
| U | (590°C)/S (590°C) | 75,5 | %/95,2 % | 73,3 | %/95,7 % |
| U | (600°C)/S (600°C) | 79,4 | %/94,6 % | 78,6 | %/95,0 % |
| Performance am Splitt | | | | | |
| (H₂O/EB = 1,25 kg/kg; 1 bar) | | | | | |
| U | (560°C)/S (560°C) | 31,2 | %/97,4 % | 26,6 | %/97,7 % |
| U | (580°C)/S (580°C) | 44,6 | %/96,7 % | 38,0 | %/97,3 % |
| U | (600°C)/S (600°C) | 57,9 | %/95,9 % | 50,1 | %/96,7 % |
| U | (610°C)/S (610°C) | 65,2 | %/95,5 % | 54,2 | %/96,4 % |
| U | (630°C)/S (630°C) | 74,3 | %/93,5 % | 65,9 | %/95,3 % |

## Patentansprüche

1. Katalysator, enthaltend Eisenoxid und 5 bis 40 Gew.-% Kalium, berechnet als K₂O, **dadurch gekennzeichnet, daß** zur Herstellung des Katalysators ein Eisenoxid, das durch Sprühröstung einer Eisensalzlösung erhalten wurde, eingesetzt wird, wobei das Eisenoxid einen Restgehalt an Chlor von 800 bis 1500 ppm aufweist.

2. Katalysator nach Anspruch 1, **dadurch gekennzeichnet, daß** das eingesetzte Eisenoxid durch Sprühröstung einer salzsauren eisenchloridhaltigen Lösung nach dem Ruthner-Verfahren gewonnen wurde.

3. Katalysator nach Anspruch 1 oder 2, **dadurch gekennzeichnet, daß** das eingesetzte Eisenoxid ein Stampfgewicht im Bereich von 0,6 bis 1 g/cm³ und eine spezifische Oberfläche im Bereich von 1 bis 10 m²/g aufweist.

4. Katalysator nach einem der Ansprüche 1 bis 3, **dadurch gekennzeichnet, daß** das Porenvolumen des Katalysators mindestens 0,2 cm³/g beträgt.

5. Katalysator nach einem der Ansprüche 1 bis 4, **dadurch gekennzeichnet, daß** der mittlere Porendurchmesser (Medianwert) des Katalysators mindestens 0,3 µm beträgt.

6. Katalysator nach einem der Ansprüche 1 bis 5, enthaltend zusätzlich mindestens einen Promotor, ausgewählt aus Verbindungen der Gruppe Mg, Ca, Ce, V, Cr, Mo, W.

7. Katalysator nach einem der Ansprüche 1 bis 6, **dadurch gekennzeichnet, daß** mindestens ein Teil der Kaliumverbindung oder ein Teil der Promotoren der Eisensalzlösung für die Sprühröstung zugesetzt wurde.

8. Katalysator nach einem der Ansprüche 1 bis 7, **dadurch gekennzeichnet, daß** der Anteil des durch Sprühröstung einer Eisensalzlösung erhaltenen Eisenoxides 10 bis 100 Mol.-% bezogen auf alle eingesetzten Eisenverbindungen beträgt.

9. Verfahren zur Herstellung eines Katalysators gemäß einem der Ansprüche 1 bis 8, **dadurch gekennzeichnet, daß** man ein durch Sprühröstung einer wässrigen Eisenlösung erhaltenes Eisenoxid einsetzt.

10. Verfahren nach Anspruch 9, **dadurch gekennzeichnet, daß** das trockene Katalysatorpulver zu mechanisch stabilen Formkörpern tablettiert oder unter Zugabe von Wasser zu Strängen extrudiert wird.

11. Verfahren zur Dehydrierung von Ethylbenzol zu Styrol, **dadurch gekennzeichnet, daß** die Dehydrierung in Gegenwart eines Katalysators gemäß einem der Ansprüche 1 bis 8 durchgeführt wird.

## Claims

1. A catalyst comprising iron oxide and 5 to 40% by weight of potassium, calculated as K₂O, and prepared using an iron oxide obtained by spray roasting an iron salt solution, the iron oxide having a residual chlorine content of from 800 to 1500 ppm.

2. The catalyst according to claim 1, wherein the iron oxide employed has been obtained by spray roasting a hydrochloric acid solution containing iron chloride by the Ruthner process.

3. The catalyst according to claim 1 or 2, wherein the iron oxide employed has a tamped weight in the range from 0.6 to 1 g/cm³ and a specific surface area in the range from 1 to 10 m²/g.

4. The catalyst according to any one of claims 1 to 3, wherein the pore volume of the catalyst is at least 0.2 cm³/g.

5. The catalyst according to any one of claims 1 to 4, wherein the median pore diameter of the catalyst is at least 0.3 µm.

6. The catalyst according to any one of claims 1 to 5, which additionally comprises at least one promoter selected from compounds from the group consisting of Mg, Ca, Ce, V, Cr, Mo and W.

7. The catalyst according to any one of claims 1 to 6, wherein at least some of the potassium compound or at least some of the promoter was added to the iron salt solution for the spray roasting.

8. The catalyst according to any of claims 1 to 7, wherein the proportion of the iron oxide obtained by spray roasting an iron solution is from 10 to 100mol%, based on all the iron compounds employed.

9. A process for the preparation of a catalyst according to any one of claims 1 to 8, which comprises using an iron oxide obtained by spray roasting an aqueous iron solution.

10. The process according to claim 9, wherein the dry catalyst powder is tabletted to give mechanically stable moldings or is extruded with addition of water.

11. A process for the dehydrogenation of ethylbenzene to styrene, which comprises carrying out the dehydrogenation in the presence of a catalyst according to any one of claims 1 to 8.

## Revendications

1. Catalyseur contenant de l'oxyde de fer et 5 à 40 % en poids de potassium, calculés sous la forme de K₂O, **caractérisé en ce que**, pour la préparation du catalyseur, on met en oeuvre un oxyde de fer qui a été obtenu par grillage sous pulvérisation d'une solution de sel de fer, l'oxyde de fer présentant une teneur résiduaire en chlore de 800 à 1500 ppm.

2. Catalyseur suivant la revendication 1, **caractérisé en ce que** l'oxyde de fer mis en oeuvre a été obtenu par grillage sous pulvérisation d'une solution chlorhydrique contenant du chlorure de fer conformément au procédé de Ruthner.

3. Catalyseur suivant la revendication 1 ou 2, **caractérisé en ce que** l'oxyde de fer mis en oeuvre présente une densité après damage de l'ordre de 0,6 à 1 g/cm³ et une surface spécifique de l'ordre de 1 à 10 m²/g.

4. Catalyseur suivant l'une des revendications 1 à 3, **caractérisé en ce que** le volume poreux du catalyseur est au moins de 0,2 cm³/g.

5. Catalyseur suivant l'une des revendications 1 à 4, **caractérisé en ce que** le diamètre moyen des pores (valeur médiane) du catalyseur est d'au moins 0,3 µm.

6. Catalyseur suivant l'une des revendications 1 à 5, contenant en supplément au moins un promoteur, choisi parmi des composés du groupe Mg, Ca, Ce, V, Cr, Mo, W.

7. Catalyseur suivant l'une des revendications 1 à 6, **caractérisé en ce qu'**au moins une partie du composé de potassium ou une partie des promoteurs a été ajoutée à la solution de sel de fer pour le grillage sous pulvérisation.

8. Catalyseur suivant l'une des revendications 1 à 7, **caractérisé en ce que** la partie de l'oxyde de fer obtenue par grillage sous pulvérisation d'une solution de sel de fer est de 10 à 100 % molaires par rapport à tous les composés de sel mis en oeuvre.

9. Procédé de préparation d'un catalyseur suivant l'une des revendications 1 à 8, **caractérisé en ce qu'**on met en oeuvre un oxyde de fer obtenu par grillage sous pulvérisation d'une solution aqueuse de fer.

10. Procédé suivant la revendication 9, **caractérisé en ce que** la poudre de catalyseur sèche est comprimée en corps façonnés mécaniquement stables ou est, avec addition d'eau, extrudée en extrudés.

11. Procédé de déshydrogénation d'éthylbenzène en styrène, **caractérisé en ce que** la déshydrogénation est effectuée en présence d'un catalyseur suivant l'une des revendications 1 à 8.
